# EUROPÄISCHE PATENTSCHRIFT

(11) **EP 1 150 680 B1**
(45) Veröffentlichungstag und Bekanntmachung des Hinweises auf die Patenterteilung: **12.01.2005**
(21) Anmeldenummer: 00902638.6
(22) Anmeldetag: 28.01.2000
(51) Int. Cl.: A61K 31/506, A61P 9/10

(54) **VERWENDUNG VON MOXONIDIN ZUR BEHANDLUNG NACH HERZINFARKT**
USE OF MOXONIDINE FOR POSTMYOCARDIAL INFARCTION TREATMENT
UTILISATION DE MOXONIDINE POUR LE TRAITEMENT POST-INFARCTUS DU MYOCARDE

(30) Priorität: 01.02.1999 DE 19903780
(43) Veröffentlichungstag der Anmeldung: 07.11.2001
(73) Patentinhaber: Solvay Pharmaceuticals GmbH, 30173 Hannover (DE)
(72) Erfinder: SCHOEMAKER, Regina, Gertruida, NL-3042 CG Rotterdam (NL)
(74) Vertreter: Gosmann, Martin, Dr.
(86) Internationale Anmeldenummer: PCT/EP2000/000655
(87) Internationale Veröffentlichungsnummer: WO 2000/045820

(56) Entgegenhaltungen:
- EP-A- 0 317 855
- WO-A-97/46241
- LADA W: "[Imidazoline receptors and use of drug blocking receptors I1]. Receptory imidazolinowe i stosowanie lekow blokujacych receptory l1." POLSKI MERKURIUSZ LEKARSKI, (1996 AUG) 1 (2) 124-5. REF: 10 , XP000886659
- WENZEL, RENE ROLAND ET AL: "I1-Imidazoline agonist moxonidine decreases sympathetic nerve activity and blood pressure in hypertensives." HYPERTENSION (DALLAS), (DEC., 1998) VOL. 32, NO. 6, PP. 1022-1027. , XP000886610
- LEPRAN, ISTVAN ET AL: "Effect of moxonidine on arrhythmias induced by coronary artery occlusion and reperfusion" J. CARDIOVASC. PHARMACOL. (1994), 24(SUPPL. 1), S9-S15 , XP000886614

## Beschreibung

Die vorliegende Erfindung betrifft die Verwendung von 4-Chlor-5-[(4,5-dihydro-1H-imidazol-2-yl)-amino]-6-methoxy-2-methylpyrimidin (= Moxonidin) und dessen physiologisch verträglichen Säureadditionssalzen zur Behandlung von Herzinfarkt-bedingten Schädigungen des Myokards und zur Herstellung von für diese Behandlung geeigneten Arzneimitteln.

Der Erfindung liegt die Aufgabe zugrunde, pharmazeutische Zubereitungen bereitzustellen, welche einen günstigen, die Genesung und/oder Rehabilitation fördernden Einfluß auf den myokardialen Zustand von Herzinfarkt-Patienten ausüben und somit zur Behandlung von Herzinfarkt-bedingten Schädigungen des Myokards im Rahmen der Myokardinfarkt- und/oder Postmyokardinfarkt-Behandlung geeignet sind.

Erfindungsgemäß werden zur Herstellung von pharmazeutischen Zubereitungen zur Behandlung von Herzinfarkt-bedingten Schädigungen des Myokards, 4-Chlor-5-[(4,5-dihydro-1H-imidazol-2-yl)-amino]-6-methoxy-2-methylpyrimidin der Formel I 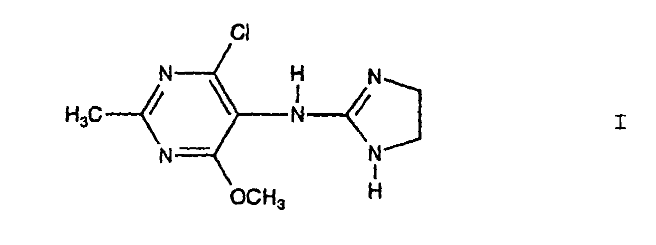 und dessen physiologisch verträgliche Säureadditionssalze verwendet.

Als physiologisch verträgliche Säureadditionssalze des Moxonidins eignen sich Salze mit anorganischen Säuren, beispielsweise Halogenwasserstoffsäuren, oder mit organischen Säuren, beispielsweise niederen aliphatischen Mono- oder Dicarbonsäuren wie Essigsäure, Fumarsäure oder Weinsäure oder aromatischen Carbonsäuren wie z. B. Salicylsäure.

Die erfindungsgemäß eingesetzten Verbindungen fallen unter den Umfang von in der deutschen Patentanmeldung Nr. 28 49 537 beschriebenen 5-[(2-Imidazolin-2-yl)-amino]-pyrimidin-Derivaten mit blutdrucksenkenden Eigenschaften, und sind aus dieser Patentanmeldung bekannt. Moxonidinhaltige pharmazeutische Zubereitungen sind als Antihypertensiva unter dem Markennamen Physiotens® im Handel erhältlich und werden medizinisch als Antihypertensivum eingesetzt. Die Verbindungen können auf an sich bekannte Weise nach den in der vorgenannten Patentanmeldung beschriebenen Verfahren oder analog zu diesen Verfahren hergestellt werden.

Es wurde nun überraschenderweise gefunden, daß Moxonidin und seine physiologisch verträglichen Säureadditionssalze einen günstigen, die Genesung und/oder Rehabilitation fördernden Einfluß auf den myokardialen Zustand nach erlittenem Herzinfarkt ausüben und somit zur Behandlung von Herzinfarkt-bedingten Schädigungen des Myokards bei Menschen und größeren Säugetieren geeignet sind.

Unter einem Herzinfarkt (Myokardinfarkt) wird im allgemeinen eine Nekrose eines umschriebenen Herzmuskelareals infolge anhaltender völliger Unterbrechung oder kritischer Herabsetzung der Blutversorgung dieses Areals verstanden. Neben allgemeinen therapeutischen Maßnahmen (Analgesie und Sedierung, Sauerstoffgabe, Bettruhe und Diät) findet beim akuten Myokardinfarkt insbesondere eine thrombolytische bzw. fibrinolytische Therapie mit dem Ziel statt, durch Reperfusion des ischämischen Areals möglichst viel (primär) ischämisches Myokard vor dem endgültigen Zelltod (d. h. definitive Nekrose) zu bewahren und somit die Infarktgröße auf ein möglichst kleines Areal zu begrenzen. Weitere (unterstützende) Maßnahmen können zur Verbesserung des Myokardzustandes, insbesondere im Bereich des Infarktareals, sowohl in der akuten Phase des Myokardinfarkts als auch in der Postmyokardinfarkt-Phase beitragen.

Die erfindungsgemäß zur Behandlung von Herzinfarkt-bedingten Schädigungen des Myokards eingesetzten Verbindungen eignen sich hierbei generell zur Anwendung im Rahmen der Behandlung eines Myokardinfarktes. Sie können daher bereits bei der Behandlung eines akuten Myokardinfarktes und insbesondere aber im Rahmen einer Postmyokardinfarkt-Behandlung sowohl bei Patienten mit einer bereits erfolgten fibrinolytischen Behandlung als auch bei Patienten ohne eine solche Lyse eingesetzt werden. Bei Postinfarkt-Patienten mit Lyse wirkt die Behandlung mit den erfindungsgemäß eingesetzten Verbindungen insbesondere auch prophylaktisch gegen die Ausbildung einer myokardial bedingten Herzinsuffizienz (Myokardinsuffizienz). Dies gilt auch für solche Patienten, die bereits mit β-Adrenozeptoren-Blockern behandelt wurden.

Postinfarkt-Patienten, die keine Lyse erfahren haben, gehen in die chronische Phase des Herzinfarktes über. Für den Postinfarkt-Patienten im chronischen Stadium ist es von besonderer Bedeutung, daß das sympathische Nervensystem (SNS) eine wichtige Rolle in der kardiovaskulären Regulation spielt. So ist die sympathische Stimulation der vorrangige Mechanismus zur Erhöhung des kardialen Ausstoßes, da diese Stimulation sowohl eine Erhöhung der myokardialen Kontraktionskraft als auch der Herzfrequenz bewirkt. Der akute Herzinfarkt (Myokardinfarkt) führt unter anderem zu einer Aktivierung des SNS zur Aufrechterhaltung des Perfusionsdruckes und der Gewebeperfusion. Diese Akutsituation kann sich zu einer mehr chronischen Phase entwickeln, in der die sympathische Aktivierung zur Hypertrophie und zu Umbauprozessen (Remodeling) des verschonten Myokards beiträgt. Dieser Vorgang kann jedoch über seinen erwünschten Grad hinausgehen und die fortbestehende SNS-Aktivierung kann aus verschiedenen Gründen schädlich werden:
1) Eine chronische Aktivierung des zentralen sympathischen Nervensystems ist im Hinblick auf die Progression der Herzinsuffizienz als ungünstig zu beurteilen. Bei anhaltender adrenerger Stimulation kommt es am Herzen kompensatorisch zu einer Verringerung adrenerger Rezeptoren. Die Folge dieses Schutzmechanismus des Herzens gegen dauerhaft erhöhte Katecholamin-Spiegel ist jedoch eine bedeutsame Beeinträchtigung der Regulation von Herzfrequenz und Herzkraft über das autonome Nervensystem.
2) Die SNS-Stimulation erhöht den Gefäßtonus und somit die Nachlast des Herzens.
3) Erhöhte, zirkulierende Katecholamin-Spiegel induzieren fokale Nekrose im Herzen und tragen zur Entstehung der kardialen Hypertrophie bei.
4) Erhöhte Plasma-Katecholaminspiegel tragen zur ungünstigen Erhöhung der Herzfrequenz und Entstehung mitunter lebensbedrohlicher Herzarrhythmien bei.

Die Verhinderung bzw. Unterbindung einer zu starken sympathischen Aktivierung kann daher eine wünschenswerte Strategie zur Behandlung von Herzinfarkt-Patienten, insbesondere auch zur prophylaktischen Behandlung gegen eine Progression der Herzschwäche nach Myokardinfarkt, darstellen.

Es wurde nun überraschenderweise gefunden, daß das erfindungsgemäß zur Myokardinfarkt- und/oder Postmyokardinfarkt-Behandlung eingesetzte Moxonidin sich durch einen überraschenden, die Genesung und/oder Rehabilitation fördernden, günstigen Einfuß auf den funktionellen Zustand des Myokards von Herzinfarktpatienten, insbesondere von Postmyokardinfarkt-Patienten im chronischen Stadium, auszeichnet. Moxonidingabe nach Myokardinfarkt bewirkt eine Verminderung des Herzgewichtes und eine Senkung der sympathischen Aktivierung, gemessen über die Plasma-Noradrenalin-Spiegel. Moxonidin eignet sich somit zur Verminderung von übermäßiger kardialer Hypertrophie, insbesondere in späteren Phasen der Postmyokardinfarkt-Behandlung. Moxonidin senkt weiterhin die Plasma-Noradrenalin-Spiegel, wodurch die sympathische Aktivierung nach Myokardinfarkt wirkungsvoll normalisiert werden kann.

Zur erfindungsgemäßen Behandlung von Herzinfarkt-bedingten Schädigungen des Myokards können Moxonidin und seine physiologisch verträglichen Säureadditionssalze in üblichen pharmazeutischen Zubereitungen oral, intravenös oder auch transdermal verabreicht werden.

So können Moxonidin und seine physiologisch verträglichen Säureadditionssalze in einer, die Genesung und/oder Rehabilitation des myokardialen Zustandes fördernd wirksamen Menge zusammen mit üblichen pharmazeutischen Hilfs- und/oder Trägerstoffen in festen oder flüssigen pharmazeutischen Zubereitungen enthalten sein. Als Beispiele fester Präparate, die zur direkten oder verzögerten Wirkstoff-Freisetzung formuliert sein können, seien oral applizierbare Präparate wie Tabletten, Dragees, Kapseln, Pulver oder Granulate genannt, oder auch Supporitorien und Pflaster (Transdermale Therapeutische Systeme). Diese festen Präparate können pharmazeutisch übliche anorganische und/oder organische Trägerstoffe wie z. B. Milchzucker, Talkum oder Stärke neben pharmazeutisch üblichen Hilfsmitteln, beispielsweise Gleitmitteln oder Tablettensprengmitteln, enthalten. Im Falle von Pflastern wird der Wirkstoff in einem Wirkstoffreservoir, insbesondere z.B. einer Wirkstoffmatrix (z.B. eine Polymermatrix) untergebracht. Flüssige Präparate wie Lösungen, Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfsund/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden. Pflaster bzw. Transdermale Therapeutische Systeme können in der üblichen Weise z.B. aus Abdeckfolie, Wirkstoffreservoir (selbsthaftend oder mit zusätzlicher Adhäsivschicht) und Abziehfolie sowohl als matrixgesteuerte als auch membrangesteuerte (d.h. mit zusätzlicher Kontrollmembran ausgerüstete) Systeme aufgebaut werden.

### Beschreibung der Tests und Ergebnisse

Die günstigen Wirkungen von Moxonidin in der Myokardinfarkt- und insbesondere in der Postmyokardinfarkt-Behandlung können in Standardtests zur Bestimmung von pharmakologischen Indikatoren für die Wirkung von Substanzen auf solche Faktoren, die den funktionellen Zustand des Myokards nach erlittenem Herzinfarktes beeinflussen, nachgewiesen werden. Ein geeignetes Tiermodell für den Nachweis von Wirkungen auf Faktoren, die den funktionellen Zustand des Myokards insbesondere im chronischen Stadium des Herzinfarktes beeinflussen, sind z. B. Wistar-Ratten mit chronischem Myokardinfarkt (MI).

Für dieses Tiermodell (MI-Ratten) wurde gefunden, daß die Plasma-Noradrenalin-Spiegel nach Myokardinfarkt akut ansteigen. In fortgeschrittenen Phasen der Herzinsuffizienz kann der Plasma-Noradrenalin-Spiegel weiter ansteigen. Die sorgfältige Untersuchung der MI-Ratten ließ erkennen, daß sogar noch drei Wochen nach dem Infarkt, d. h. nach der sogenannten Heilungsperiode, die Herzfrequenz (gemessen in vivo an uneingeschränkten, wachen Ratten) erhöht zu sein scheint, während der verbliebene Plasma-Noradrenalin-Spiegel noch um etwa 50 % höher als in Sham-Ratten (siehe weiter unten: scheinoperierte Ratten ohne Ligation der Koronararterie) lag. Weiterhin wurde im Zentralnervensystem dieser Tiere ein gesteigerter Metabolismus im paraventrikulären Hypothalamus und im Locus coeruleus beobachtet, in welchem die sympathischen Auswirkungen auf die Peripherie reguliert werden. Verhaltensstudien zeigten eine erhöhte Angst in den Infarkt-Ratten. Die vorstehenden Beobachtungen zeigen somit, daß in diesem Infarkt-Ratten-Modell eine chronisch erhöhte sympathische Aktivierung vorlag.

### Versuchstiere und Dosierung:

Die nachfolgenden Versuche wurden an männlichen Wistar-Ratten (270 bis 320 g, Harlan Zeist, Niederlande) durchgeführt. Die Ratten wurden mit einem 12-Stunden Licht/Dunkelzyklus und Standardrattenfutter sowie Wasser, jeweils verfügbar ad libitum, gehalten. Die Tiere wurden einer koronaren Arterienligation (MI-Ratten) oder einer Scheinoperation ohne Ligation (Sham-Ratten) unterworfen. Nach 24 Stunden wurden die MI-Ratten zufallsverteilt und danach erfolgte eine Implantation osmotischer Minipumpen (Alzet, Modell 2001), um Moxonidin mit einer Dosis von 3 oder 6 mg/kg·Tag s.c. (subcutan) oder nur Vehikel zu verabreichen. Die Moxonidin-Behandlung wurde kontinuierlich bis zum Ende der Experimente 3 Wochen nach dem operativen Eingriff fortgeführt.

### Koronare Arterien-Ligation:

Die linke vordere absteigende Koronararterie wurde unter Pentobarbital-Anästhesie (60 mg/kg, i.p.) ligiert. Beschreibung in Kürze: Nachdem die Luftröhre intubiert war, wurde über dem vierten Interkostal-Raum ein Einschnitt in der Haut angebracht. Die aufliegenden Muskeln wurden separiert und beiseitegehalten. Die Tiere wurden dann auf positive Druckventilation gesetzt (Frequenz 65 bis 70/min, Hubvolumen 3 ml), und der Brustraum wurde durch Trennen der Interkostal-Muskeln geöffnet. Das Perikard wurde geöffnet. Das Herz wurde in situ belassen und eine 6-0 Seidennaht wurde unterhalb der linken Koronararterie in der Nähe des Ursprungs der Pulmonalarterie angelegt. Die Naht wurde festgezogen. Sham-Ratten wurden derselben Prozedur unterzogen, jedoch ohne eine wirkliche Ligation. Die Rippen wurden mit 3-0 Seide zusammengezogen. Anschließend wurden die Muskeln auf ihre ursprüngliche Position gebracht und die Haut genäht.

### Vorbereitung und Sammlung von Blutproben:

19 Tage nach dem chirurgischen Eingriff zur Erzeugung der koronaren Arterienligation wurden die Ratten wiederum mit Pentobarbital narkotisiert, und ein Katheter (PE-10, Hitze-verschweißt mit PE-50) wurde durch die femorale Arterie in der abdominalen Aorta plaziert. Der Katheter wurde subkutan zum Nacken des Tieres geführt, wo man ihn nach außen durchtreten ließ, und er wurde dort fixiert und geschlossen. Den Ratten wurden zwei Tage zur Erholung genehmigt. Am Tag der Probenentnahme wurde der Katheter mit einem Heparin-behandelten, Salin-gefüllten Schlauch verlängert und nach wenigstens 60 Minuten wurden 2 Blutproben von 1 ml entnommen. Das Blut wurde in vorgekühlten Probentöpfchen (Syringe) gesammelt, die mit 10 µl EDTA (0,1 M) vorbereitet waren. Nach dem Zentrifugieren wurde das Plasma in vorgekühlten Röhrchen gesammelt, die entweder mit 1,2 mg Gluthation oder mit 10 µl Aprotinin (100 KIU; KIU = kilo international units) gefüllt waren, um entweder Katecholamine oder den atrialen natriuretischen Faktor (ANF) zu bestimmen. Die Röhrchen wurden bei -80 °C gelagert. Die Plasmakonzentrationen von Noradrenalin, Adrenalin und Dopamin wurden durch HPLC ermittelt, während andererseits die Konzentrationen von ANF unter Benutzung eines RIA-Tests analysiert wurden.

### Messung des kardialen Kollagens:

Die Menge des interstitiellen Kollagens wurde an 6 bis 7 zufällig aus jeder experimentellen Gruppe ausgewählten Herzen bestimmt. Die Herzen wurden hierzu durch Perfusion mit 3,6 gew.-%igem Phosphat-gepuffertem Formaldehyd fixiert. Die Ventrikel wurden, nach Entfernung der Atria (Vorhöfe) und großen Gefäße, ausgehend vom Apex (Herzspitze) zur Basis in 4 Scheiben geschnitten und die Scheiben wenigstens 24 Stunden in Formaldehyd aufbewahrt. Nach der Fixierung wurden die Scheiben entwässert und in Paraffin eingebettet. Entparaffinierte 5 µm dünne Schnitte wurden 5 Min. mit 0,2 % Gew./Vol.) wäßriger Phosphormolybdänsäure, und danach 45 Min. mit 0,1 Gew.-% Sirius Rot F3BA (Firma Polysciencies Inc., Northampton, Großbritannien) in gesättigter Pikrinsäure inkubiert, dann 2 Min. mit 0,01 M Salzsäure gewaschen, entwässert, und für mikroskopische Untersuchungen in Entellan (Firma Merck, Darmstadt, Deutschland) eingebettet. Interstitial Kollagen wurde, entfernt vom Infarktbereich, im interventrikularem Septum als Sirius Rot positiver Bereich bei 40-fachem Vergrößerungsfaktor pro Herz bestimmt.

### Datenauswertung:

Die gefundenen Daten wurden als Gruppenmittel ± S.E.M. (standard error of the mean) ausgedrückt, sofern nichts anderes angegeben ist. Nur die Daten der Infarktherzen mit einem Infarktbereich umfassend den Hauptteil der freien Herzwand des linken Ventrikels wurden in die Auswertung aufgenommen, da kleinere Infarktbereiche gewöhnlich hämodynamisch voll kompensiert werden. Die Daten wurden durch eine "Einweganalyse der Varianz (ANOVA)", gefolgt von einer post-hoc-Analyse nach Bonferroni analysiert. Unterschiede in den strukturellen Parametern der Gefäße in Moxonidin-behandelten und unbehandelten Infarktherzen wurden mittels eines Student's t-Test für die beiden Gruppen unabhängig ermittelt.

### Ergebnisse:

Es wurden vier Gruppen von Ratten untersucht: 2 Gruppen Moxonidin-behandelte Infarkt-Ratten (Dosis 3 und 6 mg/kg·Tag), nichtbehandelte Infarkt-Ratten und scheinoperierte Vergleichsratten (SHAM-Ratten). Die koronare Arterienligation erzeugte einen Hauptinfarkt in der freien Wand des linken Ventrikels. Die Gesamtmortalität der Versuchstiere lag bei 29 % und unterschied sich nicht in den zwei Infarkt-Gruppen. Daten von 5 Ratten mußten bei der 6 mg/kg·Tag-Gruppe ausgeschlossen werden, da sie einen zu kleinen Infarktbereich aufwiesen. Die Ergebnisse der Tests sind für Versuche mit einer Dosis von 6 mg/kg·Tag und für Versuche mit einer Dosis von 3 mg/kg·Tag in Tabelle 1 zusammengefaßt und werden nachfolgend erläutert. Die in Tabelle 1 angegebenen Ergebnisse enthalten die Daten von Gruppen von 7 bis 14 Ratten, mit Ausnahme der Kollagenmessungen, für sich die Daten auf Gruppen von 6 bis 7 Ratten beziehen.

Obwohl die Versuchstiere zu Beginn der Tests ein ähnliches Körpergewicht besaßen, wiesen Moxonidin-behandelte Infarkt-Ratten ein geringfügig niedrigeres Körpergewicht verglichen mit den unbehandelten Infarkt-Ratten auf, und verglichen mit den Sham-Ratten jedoch ein signifikant niedrigeres Körpergewicht. Das Herzgewicht der Moxonidin-behandelten Infarkt-Ratten war signifikant niedriger als das Herzgewicht nichtbehandelter Infarkt-Ratten. Die Effekte waren im Bereich von ca. 3 bis 6 mg/kg·Tag dosisabhängig (siehe Tabelle 1). Die Daten lassen die Schlußfolgerung zu, daß eine übermäßige kardiale Hypertrophie durch Moxonidingabe unterbunden wurde.

Die neurohumorale Aktivität, gemessen anhand der Plasma-Noradrenalin- und ANF-Spiegel, war in nichtbehandelten Infarkt-Ratten signifikant erhöht. Die ANF-Plasma-Spiegel für Ratten mit Moxonidin-Behandlung waren gegenüber denen für unbehandelte Infarkt-Ratten nicht verändert. Die Plasma-Noradrenalin-Spiegel wurden durch Moxonidin-Behandlung auf etwa die Hälfte des für die Sham-Ratten gefundenen Wertes vermindert.

Die gemessenen Plasma-Noradrenalin-Spiegel waren in nichtbehandelten Infarktratten signifikant erhöht und betrugen bis zum dreifachen des Wertes der Sham-Ratten. Die Plasma-Noradrenalin-Spiegel wurden durch die Behandlung von Infarkt-Ratten mit Moxonidine in der 6 mg/kg·Tag-Gruppe auf fast die Hälfte der Werte der Sham-Ratten abgesenkt. Bei der 3 mg/kg·Tag-Gruppe waren die Plasma-Noradrenalin-Spiegel deutlich reduziert. Dies zeigt, daß die Dosen von 3 oder 6 mg/kg·Tag Moxonidin die sympathische Aktivierung nach einem Myokardinfarkt in Ratten wirksam vermindern können.

Die Ergebnisse der Messung des kardialen Kollagens sind aus der Tabelle 1 für die 3 und 6 mg/kg·Tag-Gruppen ebenfalls ersichtlich.

Die Herzfrequenz gemessen an wachen Tieren war bei den Infarkt-Ratten gegenüber Sham-Ratten deutlich erhöht. Diese Tachykardie wurde durch Moxonidingabe nicht nur verhindert, sondern die behandelten Infarkt-Ratten zeigten gegenüber den Sham-Ratten eine verlangsamte Herztätigkeit (Bradykardie).

**Tabelle 1**

| Versuchsergebnisse für scheinoperierte Vergleichstieren (SHAM), nicht-behandelte Infarkt-Ratten (INFARKT) und Moxonidin-behandelte Infarkt-Ratten (INF+MOX); Dosis 3 mg/kg·Tag und Dosis 6 mg/kg·Tag | | | | |
|---|---|---|---|---|
| | **SHAM** | **INFARKT** | **INF + MOX (3 mg)** | **INF + MOX (6 mg)** |
| Anzahl der Versuchstiere | 8 - 14 | 7 - 12 | 7 | 6 - 7 |
| Körpergewicht (g) | 333 ± 7 | 320 ± 10 | 301 ± 5 | 299 ± 9* |
| Herzgewicht (mg) | 1174 ± 37 | 1543 ± 75* | 1408 ± 104 | 1076 ± 24# |
| Herz-/Körpergewichtsverhältnis | 3,5 ± 0.1 | 4,7 ± 0.3 * | 4,7 ± 0.4* | 3,6 ± 0,2# |
| Herzfrequenz (Schläge/min.) | 351 ± 17 | 387 ± 8* | 334 ± 12# | 321 ± 6* # |
| MAP (mmHg) | 111 ± 2 | 98 ± 3* | 87 ± 3* | 100 ± 5 |
| Plasma NA-Spiegel (pg/ml) | 199 ± 30 | 578 ± 143* | 345 ± 108 | 96 ± 20# |
| Plasma ANF (pg/ml) | 38 ± 3 | 53 ± 5 | (54 ± 2, n-3) | 61 ± 8 |
| Interstitial Kollagen (%) | 1,3 ± 0,1 | 2,2 ± 0,3* | 2,4 ± 0,2* | 1,4 ± 0,1 |
| Abkürzungen: | | | | |
| MAP = Mittlerer Arterieller Druck; NA = Noradrenalin; | | | | |
| ANF = Atrialer Natriuretischer Faktor | | | | |

| | | | | |
|---|---|---|---|---|
| * = signifikant verschieden von Sham-Ratten | | | | |
| # = signifikant verschieden von nicht-behandelten Infarkt-Ratten. | | | | |

Diese Versuchsergebnisse sind ein deutlicher Hinweis darauf, daß sich durch die Verabreichung von Moxonidin im Rahmen einer Myokardinfarkt-Behandlung und insbesondere im Rahmen einer Postmyokardinfarkt-Behandlung der funktionelle Zustand des Myokards günstig beeinflussen läßt. Aus den gefundenen Plasma-Katecholamin-Spiegeln läßt sich ableiten, daß Moxonidin die sympathische Aktivierung in Infarkt-Ratten wirkungsvoll normalisieren kann. Dieses Ergebnis wird durch die Herzfrequenz-Daten (in vivo, an wachen Ratten) bestätigt, da die Herzfrequenz in Moxonidin-behandelten Ratten sogar unter den Werten für Sham-Ratten lag. Dies ist vermutlich eher auf eine chronische als auf eine akute Wirkung von Moxonidin zurückzuführen, da bei akuter Behandlung die verminderte Herzfrequenz durch einen Anstieg des mittleren arteriellen Blutdrucks begleitet wird, der bei chronischer Behandlung nicht beobachtet wurde. Die gefundenen positiven Effekte auf das Herzgewicht-/Körpergewicht-Verhältnis Hypertrophie) scheinen zwar nicht signifikant und die Messung des Interstitial Kollagens läßt allenfalls auf einen geringen Remodeling-Effekt schließen, dennoch weisen diese Ergebnisse eine erkennbare Tendenz für eine vorbeugende Wirkung von Moxonidin gegen übermäßige kardiale Hypertrophie und unerwünschtes Remodeling auf.

Die vorstehenden Versuchsergebnisse zeigen somit, daß Moxonidin und seine Säureadditionssalze einen günstigen, die Genesung und/oder Rehabilitation fördernden Einfluß auf den myokardialen Zustand nach erlittenem Herzinfarkt ausüben und somit zur Behandlung von Herzinfarkt-bedingten Schädigungen des Myokards bei Menschen und größeren Säugetieren, sowohl im Rahmen der Behandlung eines akuten Myokardinfarkts und insbesondere auch im Rahmen einer Postmyokardinfarkt-Behandlung, geeignet sind. Moxonidin kann dabei insbesondere im Rahmen einer Postmyokardinfarkt-Behandlung auch eine prophylaktische Wirkung im Hinblick auf die Progression der Herzschwäche nach Myokardinfarkt ausüben. Die zu verwendeten Dosen von Moxonidin oder seinen Säuredditionssalzen können individuell verschieden sein und variieren naturgemäß je nach Art des zu behandelnden Zustandes und der Applikationsform. Im allgemeinen liegen die Tagesdosen für die Myokardinfarkt- bzw. Postmyokardinfarkt-Behandlung beim Menschen bei oraler Applikation im Bereich von 0,05 bis 5 mg, vorzugsweise etwa 0,25 bis 3 mg. Moxonidin oder seine Säureadditionssalze können hierbei in pharmazeutischen Zubereitungen sowohl zur sofortigen als auch verzögerten, kontrollierten und/oder gesteuerten Wirkstoff-Freisetzung verabreicht werden. Hierbei versteht es sich für den Fachmann von selbst, daß Zubereitungen mit verzögerter, kontrollierter und/oder gesteuerter Wirkstoff-Freisetzung höhere Gehalte an Wirkstoff aufweisen können als Zubereitungen zur sofortigen Wirkstoff-Freisetzung.

Das folgende Beispiel soll die Herstellung einer zur Myokardinfarkt und/oder Postmyokardinfarkt-Behandlung geeigneten Moxonidin enthaltenden pharmazeutischen Zubereitung näher erläutern.

### Beispiel 1:

### Moxonidin-haltige filmüberzogene Tabletten

### Zusammensetzung:

| Tablettenkerne: | |
|---|---|
| Moxonidin | 0,025 Teile |
| Lactose | 9,575 Teile |
| Povidone USP | 0,070 Teile |
| Crospovidone USP | 0,300 Teile |
| Magnesiumstearat | 0,030 Teile |
| (Wasser | 0,750 Teile) |
| Gesamtfeststoff | 10,000 Teile |
| | |

| Filmüberzug: | |
|---|---|
| Hydroxypropylmethylcellulose | 0,156 Teile |
| 30%ige wäßrige Ethylcellulose-Dispersion | 0,480 Teile |
| (= Feststoff) | (0,144) Teile |
| Polyethylenglycol 6000 | 0,030 Teile |
| Titandioxid | 0,150 Teile |
| Talc | 0,1197 Teile |
| Rotes Eisenoxid | 0,0003 Teile |
| (Wasser | 3,864 Teile) |
| Gesamtfeststoff | 0,600 Teile |
| Gesamtmenge an Filmüberzugssuspension | 4,800 Teile |

Zum Überziehen von 10.000 Tablettenkerne zu je 100 mg Gewicht werden 4,8 kg der vorstehenden Filmüberzugssuspension eingesetzt.

### Tablettenkernherstellung:

Das Moxonidin und die Lactose wurden gemischt. Die Mischung wurde mit einer Lösung des Bindemittels Povidone in Wasser durchfeuchtet, gut durchgeknetet und das erhaltene Produkt wurde auf Horden ausgebreitet und bei einer Temperatur von ca. 50 °C bis zu einem Feuchtigkeitsgehalt von höchstens 0,5 % getrocknet. Das getrocknete Produkt wurde durch ein 0,75 mm-Sieb (Frewitt-Maschine) passiert. Nach dem Vermischen des erhaltenen Granulates mit Crospovidone und Magnesiumstearat wurden daraus Tablettenkerne mit einem Gewicht von 100 mg gepreßt, so daß jeder Tablettenkern 0,25 mg Wirkstoff enthielt.

### Herstellung der Filmüberzugssuspension:

Die Hydroxypropylmethylcellulose und das Polyethylenglycol 6.000 wurden in einem Teil des Wassers gelöst. Zu dieser Lösung wurde eine Suspension von Talc, Titandioxid und Eisenoxid in dem übrigen Wasser unter Rühren zugefügt. Die erhaltene Suspension wurde unter leichtem Rühren mit der 30%igen wäßrigen Ethylcellulose-Dispersion verdünnt.

### Filmüberziehen der Tablettenkerne:

Die Filmüberzugssuspension wurde auf die Tablettenkerne in einer Befilmungsapparatur gesprüht, während warme Luft von ca. 70 °C die Tablettenkerne auf eine Temperatur von ca. 45 °C erwärmte. Anschließend wurden die filmüberzogenen Tabletten 16 Stunden bei einer Temperatur von ca. 45 °C getrocknet.

## Patentansprüche

1. Verwendung von 4-Chlor-5-[(4,5-dihydro-1H-imidazol-2-yl)-amino]-6-methoxy-2-methylpyrimidin der Formel I 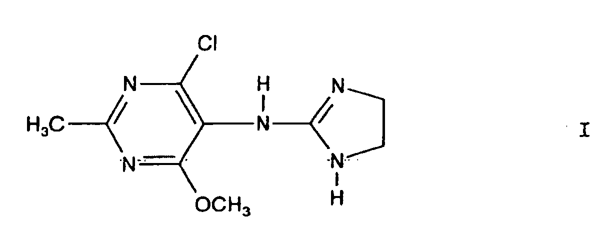 und dessen physiologisch verträglichen Säureadditionssalzen zur Herstellung von pharmazeutischen Zubereitungen zur Behandlung von Herzinfarkt-bedingten Schädigungen des Myokards.

2. Verwendung nach Anspruch 1, **dadurch gekennzeichnet, daß** man die Verbindung der Formel I gemäß Anspruch 1 oder deren physiologisch verträgliche Säureadditionssalze zur Herstellung von pharmazeutischen Zubereitungen zur Behandlung von Herzinfarkt-bedingten Schädigungen des Myokards im Rahmen der akuten Myokardinfarkt-Behandlung und/oder Postmyokardinfarkt-Behandlung verwendet.

3. Verwendung nach Anspruch 2, **dadurch gekennzeichnet, daß** man die Verbindung der Formel I gemäß Anspruch 1 oder deren physiologisch verträgliche Säureadditionssalze zur Herstellung von pharmazeutischen Zubereitungen zur Behandlung von Herzinfarkt-bedingten Schädigungen des Myokards im Rahmen der chronischen Postmyokardinfarkt-Behandlung verwendet.

## Claims

1. The use of 4-chloro-5-[(4,5-dihydro-1H-imidazol-2-yl)-amino]-6-methoxy-2-methylpyrimidine of formula I 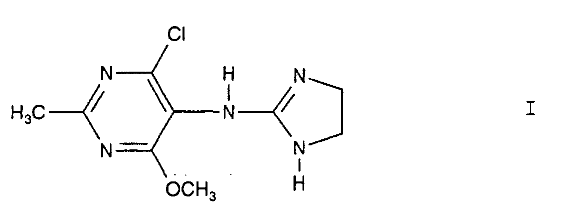 and its physiologically compatible acid addition salts for the manufacture of pharmaceutical preparations for the treatment of myocardial damage secondary to myocardial infarction.

2. The use according to Claim 1, **characterised in that** the compound of Formula I in accordance with Claim 1 or its physiologically compatible acid addition salts are used for the manufacture of pharmaceutical preparations for the treatment of myocardial damage secondary to myocardial infarction in acute myocardial infarction and/or postmyocardial infarction management.

3. The use according to Claim 2, **characterised in that** the compound of Formula I according to Claim 1 or its physiologically compatible acid addition salts are used for the manufacture of pharmaceutical preparations for the treatment of myocardial damage secondary to myocardial infarction in the management of postmyocardial infarction patients in the chronic stage.

## Revendications

1. Utilisation de 4-chloro-5-[(4,5-dihydro-1H-imidazol-2-yl)-amino]-6-méthoxy-2-méthylpyrimidine de formule I 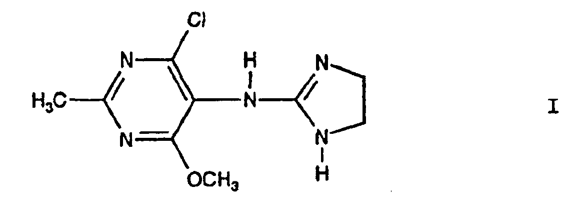 et de ses sels d'addition acides physiologiquement compatibles pour fabriquer des préparations pharmaceutiques destinées au traitement de lésions du myocarde découlant d'un infarctus du myocarde.

2. Utilisation selon la revendication 1, **caractérisée en ce que** l'on utilise le composé de formule I selon la revendication 1 ou ses sels d'addition acides physiologiquement compatibles pour fabriquer des préparations pharmaceutiques destinées au traitement de lésions du myocarde découlant d'un infarctus du myocarde dans le cadre du traitement de l'infarctus du myocarde aigu et/ou du traitement du syndrome post-infarctus du myocarde.

3. Utilisation selon la revendication 2, **caractérisée en ce que** l'on utilise le composé de formule I selon la revendication 1 ou ses sels d'addition acides physiologiquement compatibles pour fabriquer des préparations pharmaceutiques destinées au traitement de lésions du myocarde découlant d'un infarctus du myocarde dans le cadre du traitement du syndrome post-infarctus du myocarde chronique.
